# EUROPEAN PATENT APPLICATION

(11) **EP 4 163 271 A1**
(43) Date of publication of application: **12.04.2023**
(21) Application number: 21814250.3
(22) Date of filing: 26.05.2021
(51) Int. Cl.: C07D 213/24, C07D 213/00, C07D 491/02, C07D 491/056

(54) **METHOD FOR PREPARING METHYL (S)-2-AMINO-3-(4-(2,3-DIMETHYLPYRIDIN-4-YL)PHENYLPROPIONATE AND SALT THEREOF**

(30) Priority: 28.05.2020 CN 202010481658
(71) Applicant: Hangzhou Zhongmeihuadong Pharmaceutical Co., Ltd., GongShu District HangZhou, Zhejiang 310011 (CN)
(72) Inventor: HU, Haiwen, HangZhou, Zhejiang 310011 (CN); FU, Hongliang, HangZhou, Zhejiang 310011 (CN); CHEN, Fenfen, HangZhou, Zhejiang 310011 (CN); ZHOU, Xinjie, HangZhou, Zhejiang 310011 (CN)
(74) Representative: Mewburn Ellis LLP
(86) International application number: PCT/CN2021/095971
(87) International publication number: WO 2021/238965

(57) **Abstract**

The invention provides a method for preparing a key intermediate for the synthesis of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dim ethylpyridin-4-yl)phenyl)propionic acid dihydrochloride: methyl (S)-2-amino-3-(4-(2,3-dimethylpyridin-4-yl)phenylpropionate and the salt thereof. The method adopts continuous feeding mode, does not require column chromatographic purification, simplifies the procedures, reduces loss and increases yield. Furthermore, the product, i.e., salt of methyl (S)-2-amino-3-(4-(2,3-dimethylpyridin-4-yl)phenylpropionate, obtained by the purification mode of salt formation, has high purity and good storage stability. The preparation method of the invention can achieve a total yield of above 85%, a purity of the target product of above 98%, and a content of the isomer of below 0.5%. In addition, the method of the invention has good process stability, can produce quality-controlled product, and can be applied to industrial production.

## Description

### Technical Field

The invention belongs to the technical field of medicine, and particularly relates to a method for preparing an intermediate for the synthesis of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid dihydrochloride: methyl (S)-2-amino-3-(4-(2,3-dimethylpyridin-4-yl)phenylpropionate and the salt thereof.

### Background of the Invention

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid dihydrochloride, is an oral, non-peptide glucagon-like peptide 1 receptor (GLP-1R) agonist with high selectivity, and is a promising drug for treating diabetes.

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid dihydrochloride has a molecular formula of C₅₀H₄₉Cl₄N₃O₆, a molecular weight of 929.76 and the following chemical structure:

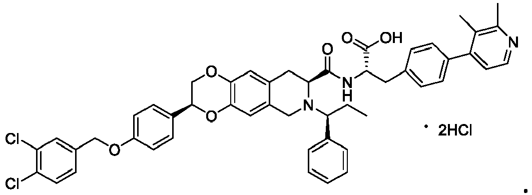

A patent for invention CN102378574B discloses (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid and the preparation method thereof, and the preparation method involves a key intermediate: methyl (S)-2-amino-3-(4-(2,3-dimethylpyridin-4-yl)phenylpropionate (compound IV). The key intermediate is obtained through a 3-step reaction route including Suzuki coupling and deprotection by using methyl (S)-3-(4-bromo-phenyl)-2-tert-butoxycarbonylamino-propionate (compound VI) as the starting material, with the following scheme:

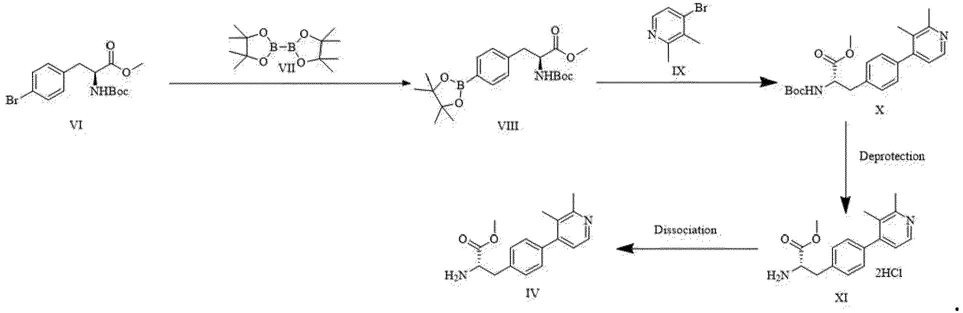

The above preparation method requires separation and purification by column chromatography, and has a total yield of only 49%; moreover, compound IX is expensive and available only in gram level on the market. Moreover, the researchers have found that compound IV is highly hygroscopic and easily to be deteriorated, it is difficult to obtain solid compound IV through conventional solvent crystallization, hence it is difficult to store, transport and industrial produce compound IV. That is to say, the prior art method not suitable for industrial production. Therefore, it is necessary to develop another synthesis route that is suitable for industrial production.

### Summary of the Invention

The invention provides a method for preparing a key intermediate for (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid dihydrochloride: methyl (S)-2-amino-3-(4-(2,3-dimethylpyridin-4-yl)phenylpropionate, and the salt thereof. The method of the invention has low cost, can achieve large-scale production and can be used in industrial production.

The invention adopts the following technical solution: compound I is used as the starting material and undergoes esterification to give compound II having higher reactivity; then compound II reacts with compound III, which is prepared beforehand, via coupling to give compound X; and compound X undergoes deprotection and then dissociation under alkaline condition to give compound IV; if necessary, compound IV may form salt again to give compound V.

The the method of the invention for preparing methyl (S)-2-amino-3-(4-(2,3-dimethylpyridin-4-yl)phenylpropionate (compound IV) and the salt thereof comprises the following steps:
(a) Reacting compound I with trifluoromethanesulfonylation agent via esterification to give compound II;
(b) Reacting compound II with compound III via coupling to give compound X;
(c) Subjecting compound X to deprotection under acidic condition to give compound IV.

The method further comprises step (d): further mixing compound IV with organic acid or inorganic acid, to give compound V after purification by salt formation, wherein x is 0.5^{~}3:

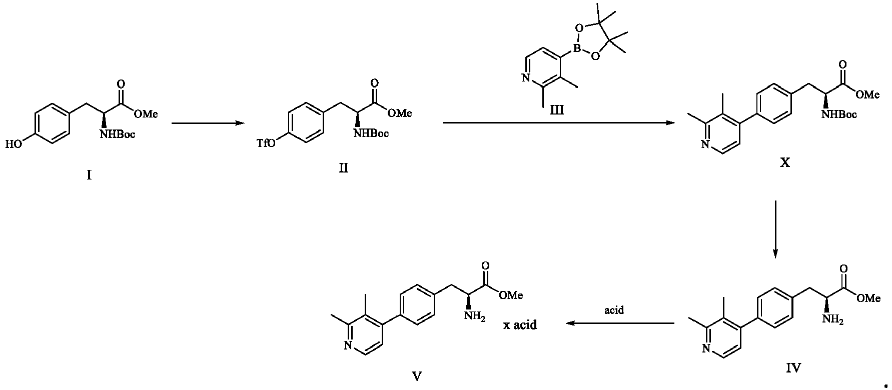

As a specific embodiment, in step (a), the solvent is one, two or more selected from the group consisting of dichloromethane, 2-methyltetrahydrofuran, tetrahydrofuran, tuluene, and xylene , and preferably dichloromethane.

As a specific embodiment, in step (a), a deacid reagent is added, wherein the deacid reagent is one selected from the group consisting of pyridine, triethylamine, dimethylaminopyridine, DBU, N-methylmorpholine, and isopropylamine, and preferably dimethylaminopyridine.

As a specific embodiment, in step (a), the trifluoromethanesulfonylation agent is one selected from trifluoromethanesulfonic anhydride, trifluoromethanesulfonyl chloride and trifluoromethanesulfonyl bromide, and preferably trifluoromethanesulfonic anhydride.

As a specific embodiment, in step (a), compound I, trifluoromethanesulfonic anhydride and the deacid reagent are fed in a molar ratio in the range of 1:1.1:1.5^{~}1:2:3.

As a specific embodiment, the reaction temperature of step (a) is in the range of -10^{~}40°C, and preferably in the range of 0^{~}20°C.

As a specific embodiment, the workup mode of step (a) is as follows: after the end of the reaction, adding water and stirring, adding diluted hydrochloric acid to adjust the pH of the system to acidic, separating and removing out the aqueous phase, concentrating the organic phase to remove solvent, adding nonpolar solvent and stirring to crystalize.

As a specific embodiment, the nonpolar solvent used in the workup of step (a) is one, two or more selected from the group consisting of petroleum ether, n-heptane, n-pentane, cyclehexane, and n-hexane , and preferably n-hexane.

As a specific embodiment, in step (b), the reaction is carried out in the presence of catalyst and base, and the catalyst is a palladium catalyst alone or a mixture of palladium catalyst and organophosphorus ligand, wherein the palladium catalyst is selected from the group consisting of Pd(OAc)₂, Pd₂(dba)₃, PdCl₂(PPh₃)₂, PdCl₂dppf, and Pd(PPh₃)₄, and the organophosphorus ligand is one, two or more selected from the group consisting of Ph₂P(CH₂)₂PPh₂(dppe), Ph₂P(CH₂)₃PPh₂(dppp), PCy₃, n-Bu₃P, P(OMe)₃, and PPh₃, and preferably a mixed system of Pd₂(dba)₃ and PCy₃; and the base is one selected from the group consisting of potassium carbonate, sodium carbonate, and cesium carbonate, and preferably potassium carbonate.

As a specific embodiment, in step (b), the solvent is a mixed system of water with one or more selected from tuluene, xylene, and n-butanol, and preferably a mixed system of water and tuluene.

As a specific embodiment, in step (b), the molar amount of the catalyst is 1^{~}5% of that of compound II, and the reaction temperature is in the range of 60^{~}120°C, and preferably in the range of 75^{~}115°C.

As a specific embodiment, in step (b), compound II and compound III are fed in a ratio in the range of 1: 1.1^{~}1: 3, and preferably in the range of 1: 1.2^{~}1: 2.

As a specific embodiment, the workup mode of step (b) is as follows: after the end of the reaction, adding diatomite to filter out unsoluble substance, separating the phases, adding diluted hydrochloric acid to the organic phase to adjust pH to acidic, separating and removing out the aqueous phase, concentrating the organic phase to dryness, adding solvent to dilute, and then directly entering into the next step of the reaction, the yield being recorded as 100%.

As a specific embodiment, in step (b), the solvent used for dilution is the reaction solvent of step (c).

As a specific embodiment, in step (c), the reaction solvent is one selected from the group consisting of tetrahydrofuran, dichloromethane/methanol, and dioxane, and preferably dichloromethane/methanol solution.

As a specific embodiment, in step (c), the acidic condition refers to adding 6^{~}12 mol/L hydrochloric acid solution.

As a specific embodiment, the workup mode of step (c) is as follows: after the end of the reaction, adding dichloromethane and purified water, stirring, adding diluted alkaline solution to adjust pH to 8^{~}10, separating and removing out aqueous phase, concentrating organic phase or directly entering into the next step of reaction, yield being recorded as 100%.

As a specific embodiment, in step (d), the acid is selected from organic acid and inorganic acid , wherein the organic acid is one selected from the group consisting of oxalic acid, tartaric acid, citric acid, succinic acid, maleic acid, malic acid, fumaric acid, glycollic acid, and hippuric acid, and preferably tartaric acid; the inorganic acid is one selected from the group consisting of phosphoric acid, sulfuric acid, and hydrobromic acid, and preferably phosphoric acid.

As a specific embodiment, in step (d), the reaction solvent is one selected from the group consisting of dichloromethane, acetone, acetonitrile, isopropanol, and tetrahydrofuran, and preferably acetone.

As a specific embodiment, in step (d), the acid is added according to the following mode: dissolving the acid in an appropriate solvent, wherein the appropriate solvent includes but is not limited to the following: methanol, ethanol, acetone, tetrahydrofuran and the like; further adding the acid in an appropriate solvent into the solution of compound IV, and then stirring the mixture.

As a specific embodiment, in step (d), in the salt formation reaction, the free base and the acid are fed at a molar ratio of free base : acid = 1:0.5^{~}1:3.5, and preferably 1: 2.0^{~}1: 2.5.

As a specific embodiment, in step (d), the salt formation temperature is in the range of -10^{~}25°C, and preferably in the range of 5^{~}20°C.

As a specific embodiment, in step (d), after the addition of the acid, the time period of stirring the mixture to crystalize is 2^{~}15 hours.

The invention achieves the following beneficial effects: the invention provides a method for preparing a key intermediate for the synthesis of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid dihydrochloride: methyl (S)-2-amino-3-(4-(2,3-dimethylpyridin-4-yl)phenylpropionate and the salt thereof. Compared with the method disclosed in the above patent, the method of the invention utilizes cheap materials, adopts continuous feeding mode in the 2-step reaction for producing methyl (S)-2-amino-3-(4-(2,3-dimethylpyridin-4-yl)phenylpropionate, does not require column chromatographic purification after each reaction, simplifies the procedures, reduces loss and increases yield. Furthermore, the product, i.e., salt of methyl (S)-2-amino-3-(4-(2,3-dimethylpyridin -4-yl)phenylpropionate, obtained by the purification mode of salt formation, has high purity and good storage stability. The preparation method of the invention can achieve a total yield of above 85%, a purity of the target product of above 98%, and a content of the isomer of below 0.5%. In addition, the method of the invention has good process stability, can produce quality-controlled product, and can be applied to industrial production.

### Detailed Description of the Invention

The invention will be further illustrated by combining the following specific examples. The following examples are used to explain the method of the invention and the core concept thereof, and for those skilled in the art, any possible change or substitution without departing from the inventive concept will fall within the protection scope of the invention. In the following examples, where the specific conditions of the experimental methods are not indicated, they are typically the conventional conditions, or are those recommended by the raw material or commodity manufactures; and the solvents without indicating the source are typically conventional solvents that are commercially available.

The detection instrument used in the present invention is:
1. NMR
   Instrument model: Bruker DMX-500 nuclear magnetic resonance instrument
2. Mass Spectrometer
   Instrument model: Agilent 6460, test conditions: ESI source

### Example 1: Preparation of compound III

Under the protection of nitrogen gas, 30 g 2, 3-dimethyl-4-chloropyridin-1-oxide, 300 ml tuluene and 50 g potassium acetate were charged in a reaction flask, stirred and mixed homogeneously, and the temperature was decreased to -5°C. 46.5 g bis(pinacolato)diboron was added, and stirred for 1 hour. Another portion of 49.8 g bis(pinacolato)diboron was added, and stirred at room temperature for 18 hours. 300 mg Pd₂dba₃ and 400 mg PCy₃ were further added, the mixture was heated to 115°C and reacted for 10 hours. After the completion of reaction, the temperature was decreased to 30°C. 420 ml n-heptane was added, and stirred for 1 hour, the mixture was filtered to give filtrate. The filtrate was warmed to 35°C, and 150 ml diluted hydrochloric acid was added and stirred for 10 minutes. The mixture was extracted with dichloromethane or ethyl acetate. 44 g compound III was obtained after concentration under reduced pressure, yield: 98%.

### Example 2: Preparation of compound II

250 ml dichloromethane and 55 g compound I were charged in a reaction flask, and stirred until dissolution. 28.6 g 2-methylpyridine was added, and the temperature was decreased to -5°C. 57.7 g trifluoromethanesulfonic anhydride was added dropwise, and the mixture was allowed to react for 1 hour. After the completion of reaction, the mixture was adjusted to pH = 2-3 with 1 N diluted hydrochloric acid. The aqueous phase was separated and removed out, the organic phase was concentrated to remove solvent, and then n-hexane was added and stirred to crystalize. 71 g compound II was obtained after filtration, yield: 90%.

### Example 3: Preparation of compound X

17 g potassium carbonate and 100 ml deionized water was charged in a reaction flask, and stirred until dissolution. 15.3 g compound III, 19.1 g compound II and 300 ml tuluene were added and stirred until the mixture was clear. Under the protection of nitrogen gas, 304 mg Pd₂dba₃ and 387 mg PCy₃ were added, the mixture was heated to 80°C and reacted for 16 hours. After the completion of reaction, the temperature was decreased to 50°C. The mixture was filtered through diatomite, and the filtrate was separated to give organic phase. The organic phase was further washed with diluted hydrochloric acid to pH 6^{~}7. The aqueous layer was separated and removed out, and the organic layer was concentrated to dryness. Then the reaction solvent of the next step was added and the mixture was directly used in the next step of reaction. Approximate 18.1 g compound X was obtained, yield being recorded as 100%.

### Example 4: Preparation of compound IV

18.1 g compound X and 100 ml dichloromethane were charged in a reaction still and stirred until dissolution. 65 ml 6 N HCl/methanol was added dropwise, and the mixture was allowed to react at room temperature for 2 hours. After the completion of reaction, 200 ml dichloromethane was added, and the mixture was adjusted to pH approximate 8 with saturated sodium bicarbonate. Organic phase was obtained after the separation of phases and reserved for use. Approximate 13.4 g compound IV was obtained, yield being recorded as 100%.

### Example 5: Preparation of compound V

To a solution of compound IV (13.4 g) as prepared in the previous step in dichloromethane, a solution of 11.5 g oxalic acid dihydrate in 27 ml ethanol was added. After the completion of addition, the mixture was stirred at room temperature to crystalize for 3 hours. 20 g compound V (dioxalate) as white solid was obtained after filtration, yield: 95%, purity: 99.1%, ee%: 98.2%. ¹H NMR (500 MHz, d6-DMSO) δ 8.37 (d, J=5.0Hz, 1H), 7.38-7.16 (m, 4H), 7.15 (s, 1H), 4.35-4.32 (m, 1H), 3.26-3.15 (m, 2H), 3.70 (s, 3H), 2.56 (s, 3H), 2.53 (s, 3H); ¹³C NMR (100 MHz, d6-DMSO) δ 169.5, 163.4(4C), 158.5, 157.8, 148.9, 145.9, 142.0, 141.8, 140.9, 138.7, 156.6, 149.7, 144.1, 137.6, 134.8, 129.5(2C), 129.4, 128.9(2C), 122.6, 53.2, 52.6, 35.7, 22.2, 15.8; It is confirmed by carbon spectrum that the structure contains 2 molecules of oxalic acid, and the molecular formula is C₁₇H₂₀N₂O₂ ·2C₂H₂O_{4∘} HRMS (ESI) Calcd. For C₁₇H₂₀N₂O₂: 285.1598 [M+H], found: 285.1604.

### Example 6: Preparation of compound V

A solution of compound IV (13.4 g) as prepared in the previous step in dichloromethane was concentrated to dryness, and then 70 g acetone was added. The mixture was stirred until dissolution. Then a solution of phosphoric acid (10.32 g) in acetone was added dropwise at 5°C. The mixture was stirred until solid appeared, and was allowed to crystalize for 3 hours. 19.9 g compound V (diphosphate) as off-white solid was obtained after filtration, yield: 90%, purity: 98.5%, ee%: 98.0%. ¹H NMR (500 MHz, d6-DMSO) δ 8.30 (d, J=5.0Hz, 1H), 7.36-7.30 (m, 4H), 7.04 (s, 1H), 4.25-4.23 (m, 1H), 3.24-3.11 (m, 2H), 3.68 (s, 3H), 2.51 (s, 3H), 2.16 (s, 3H); ¹³C NMR (100 MHz, d6-DMSO) δ 170.0, 157.3, 148.3, 145.4, 138.0, 134.7, 129.5(2C), 128.9(2C), 128.4, 122.1, 53.4, 52.5, 36.1, 23.0, 15.8; HRMS (ESI) Calcd. For C₁₇H₂₀N₂O₂: 285.1598 [M+H], found: 285.1604_{∘} With reference to the four general rules 3103 of the 2015 edition of the Chinese Pharmacopoeia, the phosphorus content is 12.3%, which proves that it contains two molecules of phosphoric acid, that the molecular formula is C₁₇H₂₀N₂O₂·2H₃PO₄.

## Claims

1. A method for preparing methyl (S)-2-amino-3-(4-(2,3-dimethylpyridin-4-yl)phenylpropionate (compound IV) and the salt thereof, **characterized in that**, the method comprises the following steps:
(a) reacting compound I with trifluoromethanesulfonylation agent to give compound II;
(b) reacting compound II with compound III to give compound X;
(c) subjecting compound IV to deprotection to give compound IV;

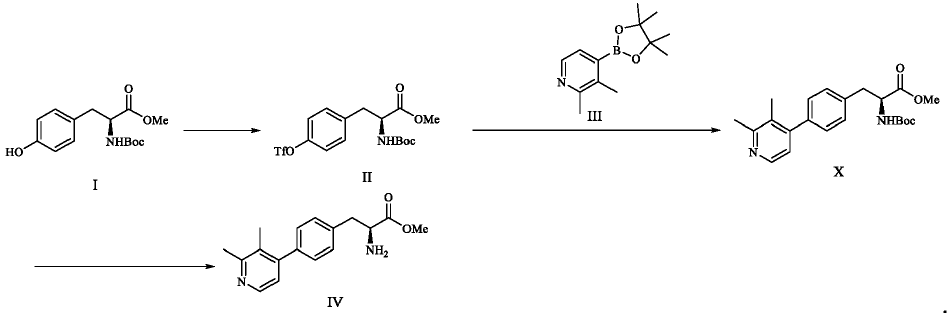

2. The method according to claim 1, **characterized in that**, the method optionally comprises step (d): mixing compound IV with organic acid or inorganic acid to form salt and then give compound V after purification, 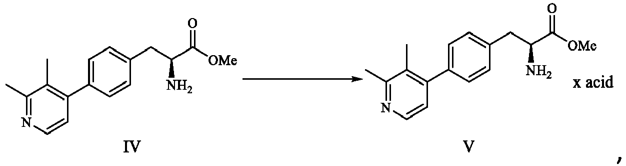 wherein x is 0.5^{~}3.

3. The method according to claim 1 or 2, **characterized in that**, in step (a), a deacid reagent is added, wherein the deacid reagent is one, two or more selected from the group consisting of pyridine, triethylamine, dimethylaminopyridine, diisopropylethylamine, DBU, N-methylmorpholine, and isopropylamine; and/or in step (a), the trifluoromethanesulfonylation agent is one selected from trifluoromethanesulfonic anhydride, trifluoromethanesulfonyl chloride and trifluoromethanesulfonyl bromide; and/or in step (a), the solvent is one, two or more selected from the group consisting of dichloromethane, tetrahydrofuran, 2-methyltetrahydrofuran, tuluene, and xylene.

4. The method according to claim 1 or 2, **characterized in that**, in step (a), compound I, trifluoromethanesulfonylation agent and the deacid reagent are fed in a molar ratio in the range of 1:1.1:1.5^{~}1:2:3, and/or the reaction temperature of step (a) is in the range of 10^{~}40°C.

5. The method according to claim 1 or 2, **characterized in that**, in step (b), the reaction is carried out in the presence of catalyst and base, and the catalyst is a palladium catalyst alone or a mixture of palladium catalyst and organophosphorus ligand, wherein the palladium catalyst is selected from the group consisting of Pd(OAc)₂, Pd₂(dba)₃, PdCl₂(PPh₃)₂, PdCl₂dppf, and Pd(PPh₃)₄, and the organophosphorus ligand is one, two or more selected from the group consisting of Ph₂P(CH₂)₂PPh₂(dppe), Ph₂P(CH₂)₃PPh₂(dppp), PCy₃, n-Bu₃P, P(OMe)₃, and PPh₃; the molar amount of the catalyst is 1^{~}5% of that of compound II; and/or the base is one of potassium carbonate, sodium carbonate, and cesium carbonate, and preferably potassium carbonate.

6. The method according to claim 1 or 2, **characterized in that**, in step (b), the solvent is a mixed system of water with one, two or more selected from the group consisting of tuluene, xylene, and n-butanol; and/or the reaction temperature of step (b) is in the range of 60^{~}120°C; and/or in step (b), compound II and compound III are fed in a molar ratio in the range of 1:1.1^{~}1:3, and preferably in the range of 1:1.2^{~}1:2.

7. The method according to claim 2, **characterized in that**, in step (d), compound IV is mixed homogeneously with solvent, a solution of organic acid or inorganic acid is added, the mixture is stirred to form salt and crystalize, and then compound V is obtained.

8. The method according to claim 2 or 7, **characterized in that**, in step (d), the acid is selected from organic acid and inorganic acid, wherein the organic acid is one selected from the group consisting of oxalic acid, tartaric acid, citric acid, succinic acid, maleic acid, malic acid, fumaric acid, glycollic acid, and hippuric acid, and preferably tartaric acid; the inorganic acid is one selected from the group consisting of phosphoric acid, sulfuric acid, and hydrobromic acid, and preferably phosphoric acid.

9. The method according to claim 2 or 7, **characterized in that**, in step (d), the molar ratio of compound IV to the acid is in the range of 1:1.1^{~}1:3, and preferably in the range of 1:2.1^{~}1:2.5, namely, preferably forming a salt comprising two acid molecules per salt molecule.

10. The method according to claim 2 or 7, **characterized in that** the salt formation temperature is in the range of -10^{~}25°C.

11. The use of compound IV according to claim 1 or 2 in preparation of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-di methylpyridin-4-yl)phenyl)propionic acid.
